# EUROPEAN PATENT SPECIFICATION

(11) **EP 0 562 041 B1**
(45) Date of publication and mention of the grant of the patent: **02.10.2002**
(21) Application number: 92904175.4
(22) Date of filing: 11.12.1991
(51) Int. Cl.: A61K 9/70

(54) **SUBSATURATED TRANSDERMAL DRUG DELIVERY DEVICE EXHIBITING ENHANCED DRUG FLUX**
VORRICHTUNG ZUR TRANSDERMALEN ABGABE VON ANNÄHERND GESÄTTIGTEN MEDIKAMENTEN MIT ERHÖHTEM MEDIKAMENTENFLUSS
DISPOSITIF D'ACHEMINEMENT TRANSDERMIQUE SOUS-SATURE DE MEDICAMENT PRESENTANT UN MEILLEUR FLUX MEDICAMENTEUX

(30) Priority: 11.12.1990 US 626685; 07.02.1991 US 652127
(43) Date of publication of application: 29.09.1993
(73) Proprietor: THERATECH, INC., Salt Lake City Utah 84108 (US)
(72) Inventor: EBERT, Charles D., Salt Lake City, UT 84103 (US); PATEL, Dinesh, Murry, UT 84107 (US); HEIBER, Werner, Salt Lake City, UT 84108 (US)
(74) Representative: Goldin, Douglas Michael
(86) International application number: PCT/US91/09408
(87) International publication number: WO 92/010231

(56) References cited:
- EP-A- 0 267 617
- WO-A-89/12470
- GB-A- 2 161 073
- GB-A- 2 185 187
- US-A- 4 820 720
- US-A- 4 855 294
- US-A- 4 906 463
- US-A- 5 059 426
- AM. J. MED. vol. 83, no. 3 , September 1987 pages 471 - 478 KORENMANN S.G. ET AL 'ANDROGEN THERAPY OF HYPOGONADAL MEN WITH TRANS SCROTAL TESTOSTERONE SYSTEMS'

## Description

### Technical Field

This invention is in the field of transdermal drug administration. More particularly it relates to a device and method that provides the drug at unexpectedly high flux.

### Background

Transdermal drug delivery devices typically comprise a drug reservoir composed of the drug and a carrier from which the drug is released by diffusion. Examples of such devices are described in "Transdermal Drug Delivery Systems," U.S. Pharmacist, pp. 49-78.

Fick's Law has classically been used to characterize the drug release kinetics of such diffusional devices. According to this law the maximum flux of drug from such a device occurs when the concentration of drug in the carrier is at saturation. Correlatively, the art teaches that the maximum flux of the drug across skin (when the skin is not a rate controlling barrier to the drug) from a given drug-carrier combination also occurs when the concentration of drug in the carrier is at saturation. Since maximum skin flux is desired with most drugs, diffusional devices have traditionally been designed to maintain saturation conditions in the carrier over the dispensing lifetime of the device.

Applicants have now unexpectedly discovered that the maximum skin flux from diffusional devices in which the drug is hydrophobic occurs when the drug is maintained below saturation in the carrier. This finding is totally contrary to the conventional wisdom followed in the transdermal drug device art. Further, applicants have employed this discovery to permit testosterone to be delivered across nonscrotal skin at therapeutically effective levels. As discussed in detail below, the art teaches that effective transdermal delivery of testosterone can only be achieved through scrotal skin.

Some prior patents have suggested in passing that while it is desirable to maintain the concentration of drug at saturation because maximum flux occurs there at, that the drug concentration could be below saturation. See for instance U.S. Pats. Nos. 4,568,343; 4,645,502; 4,816,258; 4,863,738; 4,865,848; and 4,908,027. These patents, however, fail to suggest maintenance of subsaturation levels of drug throughout the dispensing lifetime or that any increase in skin flux could be achieved with hydrophobic drugs under such conditions.

Testosterone therapy is currently indicated for treatment of male hypogonadism, anemia, breast cancer, and hereditary angioedema. It is also being considered for treating a variety of other conditions such as male osteoporosis that appear to be mediated by androgen deficiency. Traditional modalities for administering testosterone have included: intramuscular injection of long-acting testosterone esters such as the enanthate because testosterone itself is rapidly degraded by the liver if administered orally; oral administration of testosterone undecanoate, which provides systemically available testosterone; and subcutaneous implantation of fused testosterone pellets. None of these traditional modalities provides totally physiological levels or circadian patterns of testosterone and its active metabolites, dihydrotestosterone (DHT) and estradiol (E₂).

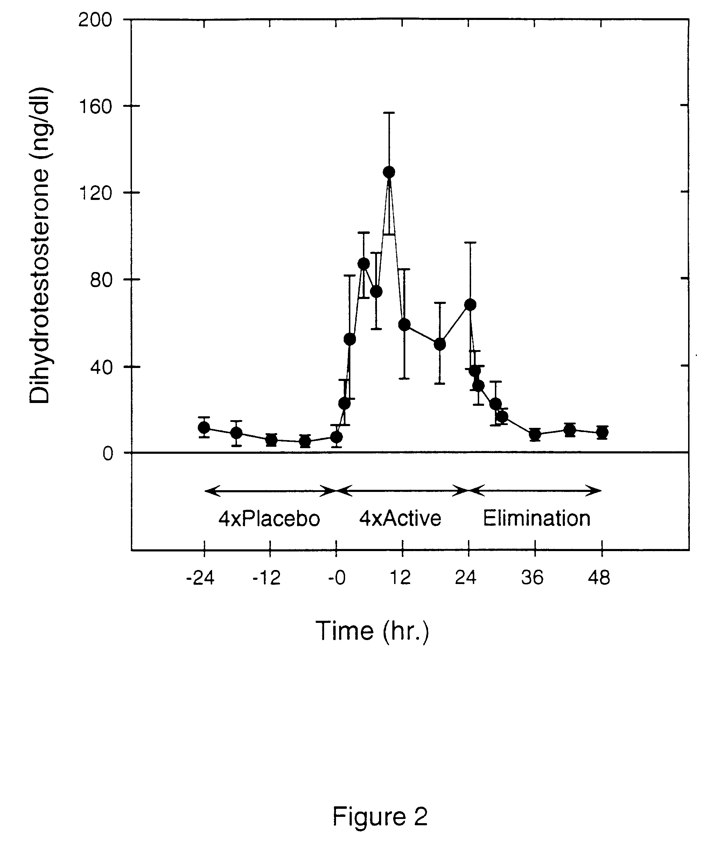

It is known that steroids, including testosterone, are absorbed through skin. However, the permeability to testosterone of skin areas that are normally used for transdermal delivery (e.g., the neck, back, chest, arms) is too low to permit delivery of the amounts of testosterone needed for therapy (typically 5-10 mg/day) through a limited area of skin. In this regard, Korenman, S.G., et al., (Am J Med (1987) 83:471-478) in an article on testosterone delivery for treating hypogonadism concluded "a more permeable skin area with a much higher absorption rate was required to provide programmed transdermal delivery to a limited area." This led Korenman et al. to select scrotal skin--which is highly permeable to testosterone--as a site for testosterone delivery. The article further describes a transscrotal delivery system developed by ALZA Corporation. U.S. 4,704,282 describes that system in detail. It consists of a polymer matrix that contains testosterone at subsaturation levels and a fabric reinforcement incorporated into the matrix that also is a limited solvent for testosterone. The patent indicates that a subsaturated matrix is used because a declining testosterone release rate is desired. The reinforcing fabric, in addition to providing a structural support function, is said to act as a secondary reservoir for testosterone which has the effect of flattening the release rate profile (see Figure 2 of the patent). While the patent states that permeation enhancers may be present in the matrix, no examples of the use of such enhancers are described. The patent gives no data on the skin flux of testosterone provided by its systems. Example 2 of the patent states that its system may be applied to nonscrotal skin, particularly the thigh, to produce "similar results" as when applied to scrotal skin. This statement is, however, contradicted by the later Korenman et al. article (which also originates from ALZA Corporation) which reports that systems applied to the thigh did not give increased blood levels of testosterone.

Ahmed, S.R., et al. (J Clin Endocrinol Metab (1988) 66:546-557) and Findlay, J.C. (J Clin Endocrinol Metab (1989) 68:369-373) report that the 60 cm² ALZA transscrotal system delivers about 3.7 mg/day and produces low-normal testosterone levels in hypogonadal men. Such dosages are believed to be somewhat less than the amount needed to mimic endogenous production (5-10 mg/day). Furthermore, since scrotal skin has a relatively high level of 5α-reductase, continuous transscrotal delivery of testosterone produces levels of DHT and DHT/testosterone ratios 4- to 5-fold greater than normal. Such abnormal levels and ratios may give rise to undesirable side effects.

GB-A-2185187 refers to transdermal drug delivery devices having subsaturated systems where the release rate tends to decrease more rapidly with time when compared with saturated systems.

EP-A-0267617 describes pharmaceutical compositions for topical administration with a penetrating-enhancing vehicle containing a lower alkanol.

WO-A-89/12470 describes transdermal delivery devices with subsaturated levels of drug which allegedly reduces irritation and prevents adhesives losing their tack.

In sum, the art teaches away from transdermally administering testosterone through nonscrotal skin because of the low permeability of such skin to testosterone. Transscrotal delivery of testosterone is taught, but such delivery is associated with high DHT and DHT/testosterone ratio levels and does not provide a level of testosterone delivery that mimics endogenous production. Further, scrotal skin is sensitive and limited in area, which may result in discomfort and poor patient acceptance of this modality of delivery.

### Disclosure of the Invention

As described above, the invention is based on the discovery that in the case of transdermal administration of hydrophobic drugs from a diffusional device, maximum skin flux is achieved at concentrations of drug in the carrier that are below saturation. In some instances the increase in flux at subsaturation is dramatically higher than at saturation. The invention thus takes the form of devices for and methods of administering hydrophobic drugs transdermally that are based on this finding.

Accordingly, in one aspect, the invention provides a device for administering by diffusion a hydrophobic drug transdermally to a patient for a prolonged period of time of at least one day, the drug having a solubility in water at room temperature of less than 50µg/ml and the device comprising:
(a) a reservoir comprising the drug dissolved in a carrier, the amount and solubility of the drug in the carrier defining a condition at the start of the period of subsaturation that is sufficient to provide a drug skin flux, over at least 60% of the period, that is at least 25% greater than the drug skin flux that would be provided if the carrier were saturated with the drug, and
(b) means for maintaining the reservoir in diffusional communication with the skin of the patient.

In another aspect, the invention provides the use of a reservoir which comprises:
a hydrophobic drug having a solubility in water at room temperature of less than 50µg/ml dissolved in a carrier, the amount and solubility of the drug in the carrier defining a condition at the start of a prolonged period of time of at least one day of subsaturation that is sufficient to provide a drug skin flux, over at least 60% of the period, that is 25% greater than the drug skin flux that would be provided if the carrier were saturated with the drug;
for the manufacture of a medicament for administering, by diffusion, the drug transdermally.

The flux may therefore be increased of a hydrophobic drug from a reservoir of the drug dissolved in a carrier that is in drug delivery communication with an area of unbroken skin of a patient. The concentration of drug in the carrier is thus below saturation at the start of the period and is maintained at subsaturation thereafter for a time sufficient to provide said increase substantially throughout the time period.

A device may be provided for administering testosterone transdermally across an area of unbroken nonscrotal skin at a flux from 5 to 30 µg/cm²/hr comprising:
(a) a reservoir comprising testosterone dissolved in a carrier, and a skin permeation enhancer, the amount and solubility of testosterone in the carrier defining a condition of subsaturation that causes enhanced permeation of testosterone through nonscrotal skin and wherein the combined permeation enhancement resulting from said condition of subsaturation and said permeation enhancer provide said flux; and
(b) means for maintaining the reservoir in diffusional communication with said area of unbroken nonscrotal skin.

### Brief Description of the Drawings

Figures 1 and 2 are graphs of the results of the tests described in Example 9.
Figures 3 and 4 are bar graphs comparing the results of the tests described in Example 9 with the prior art.
Figures 5 and 6 are graphs of the test results of Example 10.
Figures 7, 8 and 9 are graphs of data described in Example 10.

### Modes for Carrying Out the Invention

The term "drug" as used to describe the principal active ingredient of the invention device intends a biologically active compound or mixture of compounds that has a therapeutic, prophylactic and/or physiological effect on the wearer of the device. Examples of the types of drugs that may be used in the device are antiinflammatory drugs, analgesics, antiarthritic drugs, antispasmodics, antidepressants, antipsychotic drugs, tranquilizers, antianxiety drugs, narcotic antagonists, antiparkinsonism agents, cholinergic agonists, anticancer drugs, immunosuppressive agents, antiviral agents, antibiotic agents, appetite suppressants, antiemetics, anticholinergics, antihistamines, antimigrane agents, vasodilators, hormonal agents, contraceptive agents, diuretics, antihypertensive agents, cardiovascular drugs, and the like.

As used herein the term "hydrophobic" intends that the solubility of the drug in water at room temperature is <50 µg/ml. Specific examples of hydrophobic drugs are steroids such as estrogens, progestogens, testosterone, norgestrel, norethindrone acetate and medroxyprogesterone acetate.

The phrase "prolonged time period" means a period of at least about one day, usually 1-14 days, more usually 1-7 days. The term "substantially throughout" intends at least about 60% of the time period, more usually at least 80%, and preferably 100% of the period.

The term "skin flux" intends the rate of transfer of drug across skin as measured by the method of Merritt and Cooper (J Controlled Release (1984) 1:161). The units of flux are preferably µg/cm²/hr.

The term "significantly greater" that is used to characterize the increase in skin flux achieved through use of the invention will typically denote an increase in skin flux of at least about 25%, usually 25% to 400%, and more usually 50% to 200% over the skin flux provided when the carrier is saturated with the drug.

The term "nonscrotal skin" means human skin excepting the skin of the male human genitalia. It will normally denote the skin of relatively hair-free portions of the body such as the limbs, back, chest, buttocks, hips, and neck.

As used here, the term "testosterone therapy" intends treatment of any indication for which testosterone is indicated, including, without limitation, primary, secondary and other male hypogonadal states in adults and adolescents, anemia, hereditary angioedema, male contraception, male infertility disorders, post-surgical recovery, male impotence, hormone replacement in elderly males, and hypogonadal states associated with AIDS. Primary (testicular) hypogonadism disorders include Klinefelder's Syndrome, viral orchitis, and low testosterone production caused by trauma, radiation or chemotherapy, or alcohol abuse. Secondary (hypothalamic/pituitary) disorders include those associated with hypothalamic hypogonadism, suprasellar tumors, and pituitary tumors. Other male hypogonadism disorders include those associated with aging, systemic illnesses, stress, and diabetes mellitus.

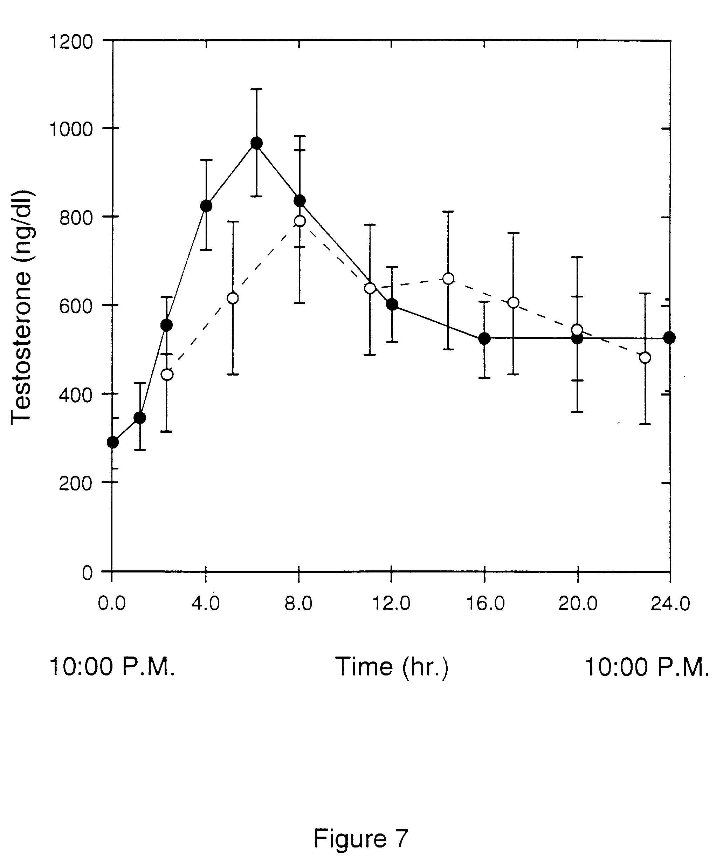

The phrase "corresponds substantially to endogenous blood levels produced by healthy young adult male humans" intends a blood level profile that closely approximates the circadian rhythm of testosterone production shown in Figure 7 of the drawings.

The devices of the invention release drug continuously by diffusion. In this mode, the driving force is the difference in drug concentration between the device reservoir and the skin and underlying tissue. The drug, which is entirely dissolved in the carrier or vehicle in the case of the present invention, permeates through the carrier to the skin. The carrier is, of course, in drug delivery (diffusional) communication with the skin--which means that it either contacts the skin directly or contacts material interposed between the carrier and the skin that provides a permeation pathway for the drug and, if present, permeation enhancer, to migrate from the reservoir to the skin. The interposed material may be homogeneous, heterogeneous, or be composed of a multiplicity of distinct layers. In any event the interposed material is permeable to the drug and preferably is not a rate-controlling barrier to diffusion (i.e., it is at least as permeable to the drug, and, if present, permeation enhancer, as the carrier).

As indicated above, the carrier or vehicle is permeable to drug. In this regard the diffusion coefficient of the drug in the carrier will usually be between 1 x 10⁻⁶ and 1 x 10⁻¹² cm²/sec, more usually between 1 x 10⁻⁷ and 1 x 10⁻¹⁰ cm²/sec. The solubility of the drug in the carrier should be such that sufficient drug is contained in the device to provide the required cumulative dose of drug, which will vary from drug to drug. At the same time, the solubility should not be so low as to require the device to be impractically large in area or thickness. In most instances, the solubility of drug in the carrier will be in the range of 1 to 500 mg/ml, more usually 1 to 200 mg/ml (measured at room temperature). The amount of drug in the carrier will normally range between 0.001 and 100 mg, more usually between 1 and 50 mg. The thickness of the reservoir will usually be about 0.01 to 5 mm, more usually 0.03 to 2 mm. The area of the device in drug delivery (diffusional) contact with the skin will usually be between about 1 and 150 cm², more usually between 5 and 40 cm².

In the case of testosterone, its solubility in the carrier should be such that sufficient testosterone is contained in the device to provide the required cumulative dose of testosterone, which will normally be in the range of 5 to 10 mg/day. At the same time, the solubility should not be so low as to require the device to be impractically large in area or thickness. The amount of testosterone in the carrier will normally range between 5 and 50 mg per unit dosage form, more usually between 10 and 20 mg. The thickness of the reservoir will usually be about 0.01 to 5 mm, more usually 0.03 to 2 mm.

The carrier may be a solid or semi-solid polymer that enables the device to be a "solid-state" device (i.e., no liquid component at room temperature). Alternatively, the carrier may be in a fluid form (e.g., liquid, gel, emulsion, suspension, and be aqueous or nonaqueous. Examples of fluid carriers that may be used are alcohols such as ethanol, alcohol-water mixtures, and low molecular weight polymers such as polyethylene glycol. Examples of solid polymeric carriers that may be used in this invention are polyacrylates, polymethacrylates, silicone polymers, polyalkyloxides, natural and synthetic rubbers and the dermatologically acceptable adhesives described in U.S. 3,934,097.

In the case of testosterone, the carrier is preferably a fluid. Examples of fluid carriers that may be used are alcohols such as ethanol, alcohol-water mixtures, and low molecular weight polymers such as polyethylene glycol. Ethanol is preferred and also provides permeation enhancement. In the case of ethanol, the carrier normally constitutes 20% to 70% by volume of the reservoir, more usually 40% to 60%, and preferably approximately 50%. Alternatively, the carrier may be a solid or semisolid matrix suxh as a pressure-sensitive adhesive.

The concentration of drug in the carrier will usually be between 10% and 80%, particularly from 20% to 80% or between 15% and 60% of saturation substantially throughout the administration period. Depending upon the nature of the carrier and other components of the reservoir (permeation enhancers), the concentration of drug relative to saturation may decrease or increase over the administration period. If the solubility of the drug in the carrier (whether modified or not by other components) remains constant over the period, the concentration relative to saturation will decrease. On the other hand, if the solubility decreases (for instance, through delivery of a permeation enhancer that also increases solubility), then the concentration relative to saturation will increase.

A permeation enhancer may be administered concurrently with the drug in order to further increase the skin flux of drug across the skin. For testosterone, an enhancer is necessary. The enhancer may also be contained within the reservoir or be administered from a separate reservoir underlying or overlying the drug reservoir. For design simplicity, when used, the enhancer will preferably be contained in the drug reservoir. Aside from the requirements that the enhancer be compatible with the drug and carrier, there are no limitations on the enhancers that may be used in the invention. Examples of enhancers known in the art are those described in U.S. Pats. Nos. 3,989,816; 4,316,893; 4,863,970; 4,764,379; 4,537,776; and EPA (Pub. No.) 272,987. A preferred enhancer for use with testosterone is a mixture of ethanol (also carrier), glycerol monooleate (GMO) and methyl laurate (ML). The amounts of each of GMO and ML in the reservoir will normally be 0.5% to 5% by volume, preferably approximately 2.5%. The amount of ethanol will be that previously described. The reservoir may also contain amounts of other materials such as gelling agents and antiirritants. Glycerin is a preferred antiirritant and may be present at 5% to 50%, preferably 20% to 30% by volume. The use of glycerin as an antiirritant is described in U.S. 4,855,294.

The skin testosterone flux provided by the invention is about 5 to 30 µg/cm²/hr, and preferably about 10 to 20 µg/cm²/hr. In contrast, the testosterone skin flux provided by conventional transdermal administration is typically less than 0.5 µg/cm²/hr. The high skin fluxes realized through the invention are a result of enhancement due to the subsaturation concentration of testosterone in the carrier and the enhancement due to the permeation enhancer.

For treating male hypogonadism it is desired to provide daily administration in a 24-hr release rate profile that mimics the endogenous diurnal testosterone production pattern. This in turn leads to a circadian rhythm in testosterone levels. Figure 7 of the drawings (open circles) shows a representative circadian rhythm of testosterone production over a one-day period. As shown, testosterone levels peak in the early morning hours and then decline to trough values in the evening.

The device of the invention may be embodied in various types of structures known in the transdermal drug delivery art. For instance, the drug reservoir, which is the most important component of the device, may comprise a simple matrix of a subsaturated solution of the drug in the carrier or be in the form of a fibrous body impregnated with the subsaturated solution of drug in the carrier. In addition to the reservoir, the device includes means for maintaining the reservoir in drug-delivery communication with the skin. Such means include a carrier which is also an adhesive, a separate basal adhesive layer underlying the reservoir, a peripheral ring of adhesive that is interconnected to the reservoir, an adhesive overlay for the reservoir, and straps. Preferably the means is either an adhesive carrier or a separate underlying adhesive layer. Preferably the device is in the form of a laminated composite.

In addition to the reservoir and affixation means, the device may further include a backing that overlies the reservoir and protects the reservoir and/or prevents back-diffusion of drug from the reservoir, one or more structural layers to provide the device with appropriate mechanical properties, and/or a release liner layer that underlies the reservoir and which is removed prior to use.

These devices may be manufactured by conventional techniques used in the transdermal drug delivery device art. For instance the drug and carrier may be mixed in the desired proportions to form a homogeneous mix and cast or otherwise applied to a backing layer, followed by lamination to a release liner layer. If a separate basal adhesive layer is desired, it may be cast onto the release liner layer prior to such lamination. As indicated above, the solubility of drug in the carrier and the size (thickness of reservoir and area in drug delivery communication with the skin) are chosen to maintain subsaturation in the reservoir over the desired dispensing lifetime of the device and provide the necessary cumulative dose of drug.

The following examples further illustrate the invention and its unique characteristics. In the following examples in vitro steady state transdermal flux across human cadaver skin was determined using the method of Merritt and Cooper, supra. Unless otherwise indicated percentages and proportions are by volume.

### Example 1

Formulations of progesterone at varying concentrations were made by mixing progesterone with the indicated ingredients and applied to cadaver skin. The transdermal fluxes for these formulations are reported in Table 1 below. The meanings of the abbreviations that appear in the table are: Gly = glycerine; GDO = glycerol dioleate; ML = methyl laurate; OA = oleic acid; GMO = glycerol monooleate.

**Table 1**

| Enhancer Systems* | | Progesterone Conc.(mg/ml) | N | Flux (µg/cm²/hr) |
|---|---|---|---|---|
| 1. | 60/22.5/10/2.5/2.5/2.5 | | | |
| | EtOH/H₂O/Gly/GDO/ML/OA | 75.0 | 8 | 2.12 ± 0.47 |
| | | | | |
| 2. | 60/22.5/10/2.5/2.5/2.5 | | | |
| | EtOH/H₂O/Gly/GDO/ML/OA | 50 | 18 | 4.51 ± 1.37 |
| | | | | |
| 3. | 60/22.5/10/2.5/2.5/2.5 | | | |
| | EtOH/H₂O/Gly/GDO/ML/OA | 25 | 3 | 5.52 ± 1.38 |
| | | | | |
| 4. | 60/22.5/10/2.5/2.5/2.5 | | | |
| | EtOH/H₂O/Gly/GMO/ML/OA | 75 | 8 | 3.35 ± 2.18 |
| | | | | |
| 5. | 60/22.5/10/2.5/2.5/2.5 | | | |
| | EtOH/H₂O/Gly/GMO/ML/OA | 50 | 18 | 7.63 ± 3.00 |
| | | | | |
| 6. | 60/22.5/10/2.5/2.5/2.5 | | | |
| | EtOH/H₂O/Gly/GMO/ML/OA | 37.5 | 6 | 8.18 ± 0.90 |
| | | | | |
| 7. | 60/22.5/10/2.5/2.5/2.5 | | | |
| | EtOH/H₂O/Gly/GMO/ML/OA | 25 | 18 | 6.37 ± 1.88 |
| | | | | |
| 8. | 60/22.5/10/2.5/2.5/2.5 | | | |
| | EtOH/H₂O/Gly/GMO/ML/OA | 10 | 3 | 1.84 ± 0.33 |

| | | | | |
|---|---|---|---|---|
| * Systems #1 - #8 were gelled by adding 2.5% (w/v) Carbopol® 1342, pHs were unadjusted (3.2 - 3.5) and the loading doses were 0.075 ml. | | | | |

In Table 1, systems 1 and 4 contain progesterone at saturation. Systems 1-3 are alike except for progesterone concentration, and systems 4-8 are alike except for progesterone concentration. The two sets of systems are alike except that one (1-3) contains GDO and the other (4-8) contains GMO. As shown by the flux data in Table 1, the flux is significantly greater in those systems (except 8) in which the progesterone is at subsaturated concentrations (systems 1, 3, 5-7) than when the progesterone is at saturation.

### Example 2

Additional progesterone systems were formulated and tested as in Example 1. The results of these tests are shown in Table 2 below. Abbreviations are as in Example 1. Progesterone was present at saturation in system 1 and below saturation in systems 2-6.

**Table 2**

| Enhancer Systems* | | Progesterone Conc. (mg/ml) | N | Flux (µg/cm²/hr) |
|---|---|---|---|---|
| 1. | 60/22.5/10/2.5/2.5/2.5 | | | |
| | EtOH/H₂O/Gly/GDO/ML/OA | 50 | 12 | 6.01 ± 1.73 |
| | | | | |
| 2. | 60/28/10/1/1 | | | |
| | EtOH/H₂O/Gly/GMO/ML | 30 | 3 | 13.03 ± 3.35 |
| | | | | |
| 3. | 60/28/10/1/1 | | | |
| | EtOH/H₂O/Gly/GMO/ML | 25 | 3 | 12.98 ± 2.06 |
| | | | | |
| 4. | 60/28/10/1/1 | | | |
| | EtOH/H₂O/Gly/GMO/ML | 20 | 9 | 15.89 ± 6.81 |
| | | | | |
| 5. | 60/28/10/1/1 | | | |
| | EtOH/H₂O/Gly/GMO/ML | 15 | 12 | 13.13 ± 1.87 |
| | | | | |
| 6. | 60/28/10/1/1/1 | | | |
| | EtOH/H₂O/Gly/GMO/ML | 10 | 5 | 11.13 ± 1.98 |

| | | | | |
|---|---|---|---|---|
| * Systems were gelled by adding 2.5% (w/v) Carbopol® 1342 and the loading doses were 0.075 ml. | | | | |

As in Example 1, the fluxes of progesterone at concentrations below saturation were significantly greater than at saturation.

### Example 3

This Example shows that the phenomenon of higher drug flux at subsaturation unexpectedly occurs only with hydrophobic drugs.

Formulations of the hydrophilic drugs oxybutynin HCl and mecamylamine HCl were prepared and tested as in Examples 1 and 2. Tables 3 and 4 below report the results of those tests. The formulations of Table 3 containing oxybutynin HCl at 40 mg/ml were saturated and the formulations of Table 4 containing 80 mg/ml of mecamylamine HCl were saturated. All other systems were at drug concentrations below saturation.

**Table 3**

| Enhancer Systems | | Oxybutyrin Conc. (mg/ml) | N | Flux (µg/cm²/hr) |
|---|---|---|---|---|
| 1. | 40/53/5/2 | | | |
| | EtOH/H₂O/Gly/GMO | 40 | 4 | 29.1 ± 11.2 |
| | | 20 | 12 | 16.9 ± 5.2 |
| | | 10 | 6 | 13.3 ± 2.6 |
| | | 5 | 3 | 5.6 ± 0.9 |
| 2. | 40/54/5/1 | | | |
| | EtOH/H₂O/Gly/GMO | 40 | 5 | 38.8 ± 18.9 |
| | | 20 | 12 | 17.5 ± 5.1 |
| | | 10 | 6 | 10.1 ± 3.3 |
| | | 5 | 3 | 8.0 ± 1.4 |
| 3. | 30/63/5/2 | | | |
| | EtOH/H₂O/Gly/GMO | 40 | 6 | 23.9 ± 10.0 |
| | | 20 | 12 | 14.8 ± 6.1 |
| | | 10 | 9 | 8.3 ± 3.8 |
| | | 5 | 3 | 2.2 ± 0.2 |
| 4. | 30/64/5/1 | | | |
| | EtOH/H₂O/Gly/GMO | 40 | 6 | 27.5 ± 13.1 |
| | | 20 | 15 | 13.6 ± 5.2 |
| | | 10 | 6 | 5.4 ± 2.5 |
| | | 5 | 3 | 1.7 ± 0.3 |

**Table 4**

| | | | Loading | | |
|---|---|---|---|---|---|
| Enhancer Systems | | Mecamylamine Conc. (mg/ml) | Dose (µl) | N | Flux (µg/cm²/hr) |
| 1. | 50/49/1 | | | | |
| | EtOH/H₂O/GMO | 80 | 400 | 3 | 534.8 ± 56.2 |
| | | 40 | 400 | 3 | 179.1 ± 51.6 |
| | | 20 | 400 | 3 | 126.7 ± 52.8 |
| 2. | 50/49/1 | | | | |
| | EtOH/H₂O/GMO | 80 | 75 | 3 | 96.5 ± 9.2 |
| | | 40 | 75 | 9 | 37.4 ± 11.0 |
| | | 20 | 75 | 3 | 25.1 ± 1.9 |
| 3. | 50/44/5/1 | | | | |
| | EtOH/H₂O/Gly/GMO | 80 | 75 | 6 | 78.4 ± 36.5 |
| | | 40 | 75 | 9 | 26.7 ± 9.5 |

The flux data of Tables 3 and 4 indicate that in each instance the drug release profile was Fickian with flux decreasing with decreasing concentration below saturation.

Similar tests were carried out on ointment and solid matrix systems containing pindolol free base as the hydrophilic drug. Again, systems exhibited classic Fickian dependence of flux on drug concentration.

### Example 4

Formulations of testosterone at saturation and below saturation were prepared and tested as in Example 1. The carrier used was EtOH/H₂O/Gly/GMO/ML in a ratio of 60/30/5/2.5/2.5. The results of these tests are shown in Table 5 below. The formulations containing 50 mg/ml testosterone were saturated, whereas the systems containing 40 mg/ml and below were subsaturated. The results are expressed in terms of cumulative permeations at 24 hr (i.e., µg/cm²) rather than as flux.

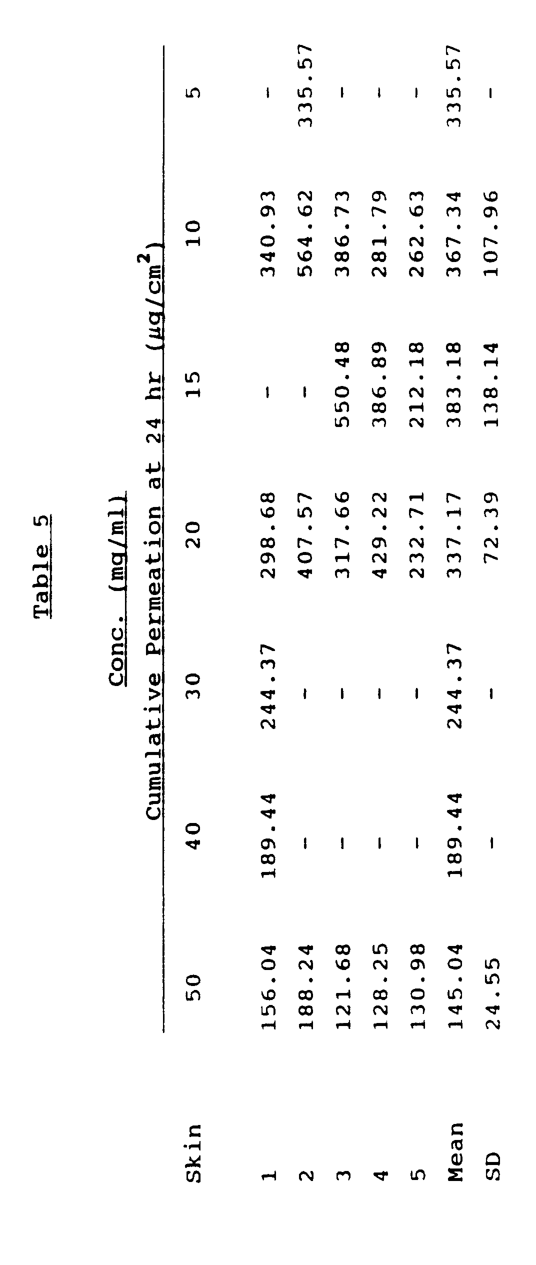

As shown, the permeation was significantly greater when the testosterone was present at subsaturation concentrations. Similar tests were carried out using the following carrier compositions:

| | | |
|---|---|---|
| EtOH/H₂O/Gly/GMO/ML/OA | - | 60/27.5/5/2.5/2.5/2.5 |
| EtOH/H₂O/Gly/GMO/ML | - | 60/33/5/1/1 |
| EtOH/H₂O/Gly/GMO/ML | - | 60/25/5/5/5 |
| EtOH/H₂O/Gly/GMO/ML | - | 50/35/5/5/5 |
| EtOH/H₂O/Gly/GMO/ML/OA | - | 50/37.5/5/2.5/2.5/2.5 |

In each instance the formulations below saturation exhibited higher permeations than the corresponding formulation at saturation.

### Example 6

Estradiol-containing matrices were prepared by mixing acrylic adhesive (National Starch Durotac® 1194), sorbitan monooleate (Arlacel® 80) and estradiol at a ratio of 80-X/20/X where X is the proportion (wt%) of estradiol. The cumulative permeation at 24 hr of estradiol from these matrices were tested as above and are reported in Table 7 below. The matrix containing 8% estradiol was saturated; the others were subsaturated.

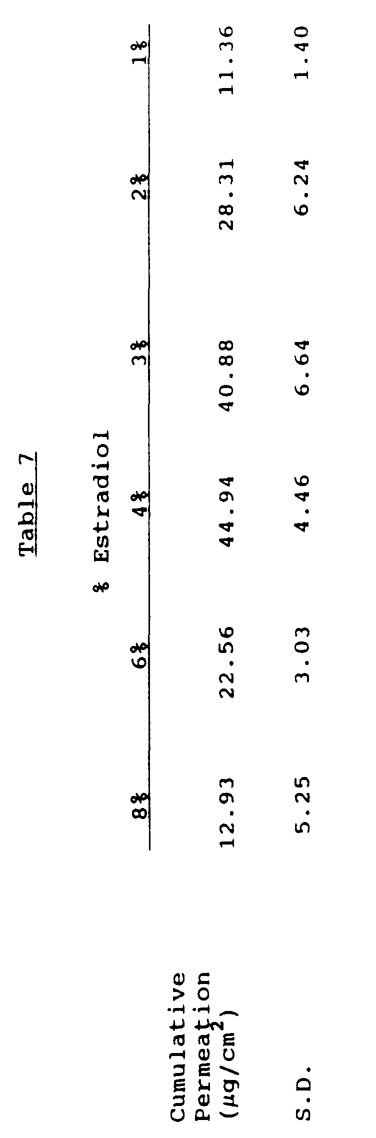

As reported in the table, the maximum permeation values observed at subsaturation were approximately three-fold that observed at saturation.

Similar tests were carried out on estradiol-containing matrices in which sorbitan monolaurate was substituted for sorbitan monooleate and in ointments using the carrier EtOH/H₂O/Gly/GMO/ML - 20/60/5/7.5/7.5. In these other estradiol formulations, maximum permeation was observed at estradiol concentrations below saturation.

### Example 7

Estradiol-containing matrices were prepared and tested as in Example 6 except that these matrices did not contain permeation enhancer (sorbitan monooleate). The cumulative permeations at 24 hr from these matrices are reported in Table 8 below. The matrix containing 8% estradiol was saturated; the others were subsaturated.

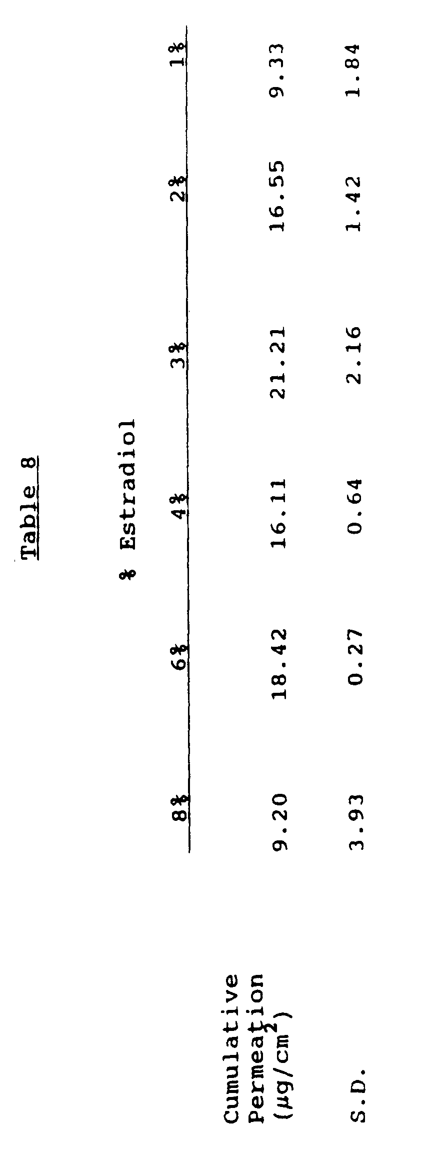

### Example 8

Norethindrone acetate-containing matrices were prepared by mixing a cross-linked acrylic adhesive (Monsanto, Gelva® 737), permeation enhancer (a 50:50 (w/w) mix of GMO and ML), and norethindrone acetate at a ratio of 80-X/15/X where X is the proportion of norethindrone acetate. Fluxes from these matrices were tested as above and are reported in Table 9 below. The matrix containing 30% norethindrone acetate was saturated; all others were subsaturated.

**Table 9**

| | % Norethindrone Acetate | | | | |
|---|---|---|---|---|---|
| | 5% | 8% | 10% | 15% | 30% |
| Flux (µg/cm²/hr) | 0.44 | 0.65 | 0.93 | 0.46 | 0.35 |

As reported, the fluxes from the subsaturated matrices were significantly higher than the flux from the matrix that contained the drug at saturation.

### Example 9

Five-layer laminated composites of the general structure described in U.S. Patent No. 4,849,224 were prepared. The layers of the composite (basal to top) were as follows:
1. 5 mil thick silicon-coated polyethylene terephthalate (Tekkote®) release liner
2. 1.5 mil thick pressure-sensitive adhesive (AR MA31 acrylic, Adhesives Research)
3. 4 mil thick peel seal disc of ethylene/vinyl acetate copolymer film (Bertek® 2216)
4. 2 mil thick microporous polyethylene film (Cotran®, 3M) and a 4-5 mil thick cavity (5 cm² surface area) filled with an ointment composed of 6.06 mg micronized testosterone, 296.88 mg ethanol, 200.10 mg water, 38.31 mg glycerin, 5.64 mg GMO, 5.27 mg ML, 0.61 mg Vitamin E, and 12.13 mg Klucel®.
5. 2 mil thick polyester/ethylene-vinyl acetate laminate (3M Scotchpak® 1012) film backing

The release liner and peel seal disc are removed for application to skin. The basal surface area of the reservoir was 5 cm².

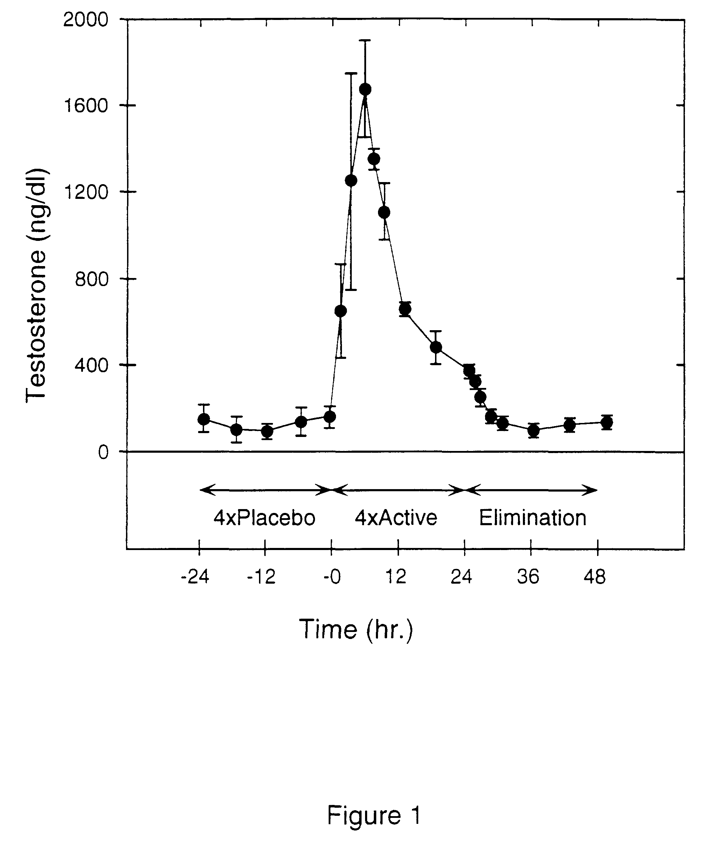

Placebo composites (four each) and the above composites (four each) were placed on the lower back skin of three hypogonadal men according to the regimen shown in Figure 1. Periodic blood samples were taken and analyzed for testosterone and DHT levels using an established radioimmunoassay.

Figures 1 and 2 show, respectively, the testosterone and DHT levels resulting from these tests.

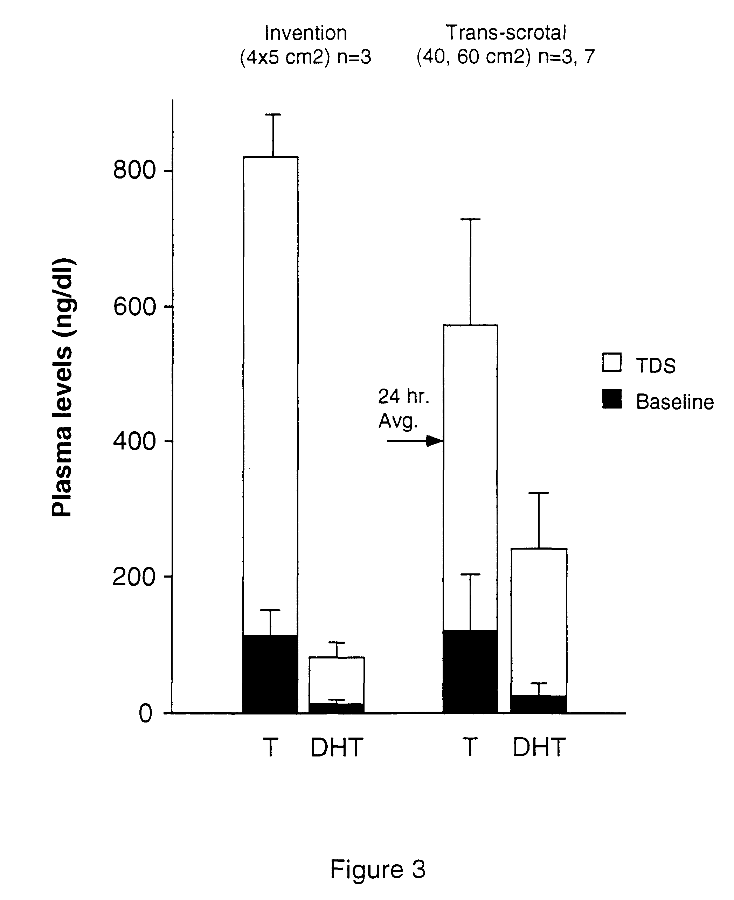

Figure 3 shows a comparison of the testosterone and DHT blood levels provided by the composite of this example and by the ALZA transscrotal system as reported by Findlay, supra. As shown, the blood levels of testosterone provided by the composite of this example are significantly higher than those provided by the transscrotal system. Correspondingly, the blood levels of DHT are significantly lower for the composite of this example as compared to the transscrotal system.

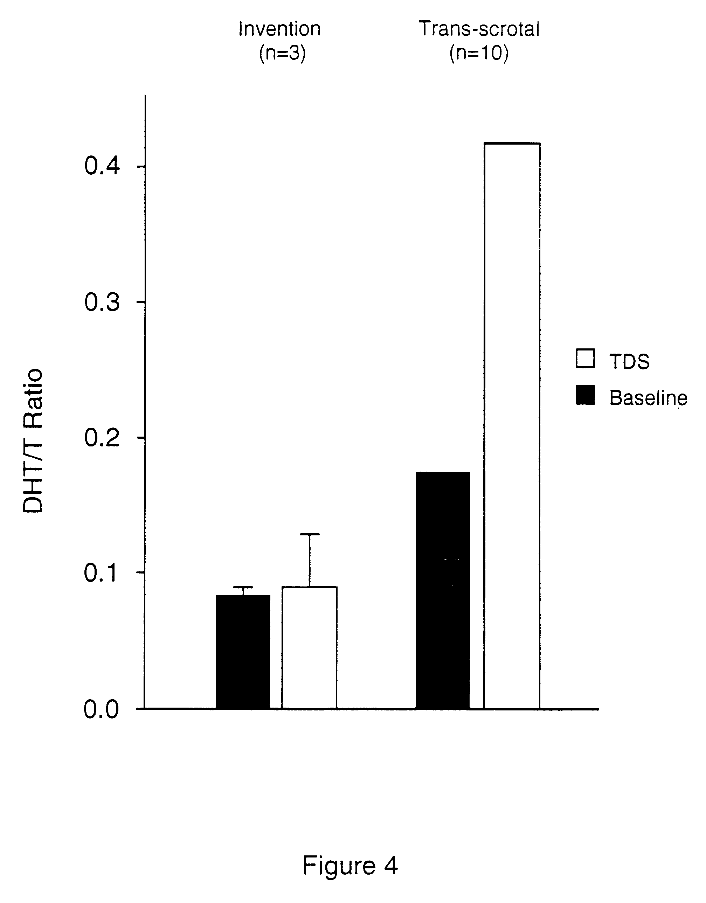

Figure 4 shows a comparison of the DHT to testosterone ratios provided by the composite of this example and the transscrotal system (again, as reported by Findlay). As shown, the ratio for the composite of this example is significantly less than the ratio for the transscrotal system.

### Example 10

A laminated composite of the same structure as that of Example 9 was prepared except that the ointment composition was: 12.4 mg testosterone, 342.40 mg ethanol, 123.40 mg water, 311.90 mg glycerin, 19.2 mg GMO, 19.9 mg ML, 27.7 mg Carbomer® 1342 and 10.2 mg 2 N NaOH. The reservoir cavity surface was 7.5 cm².

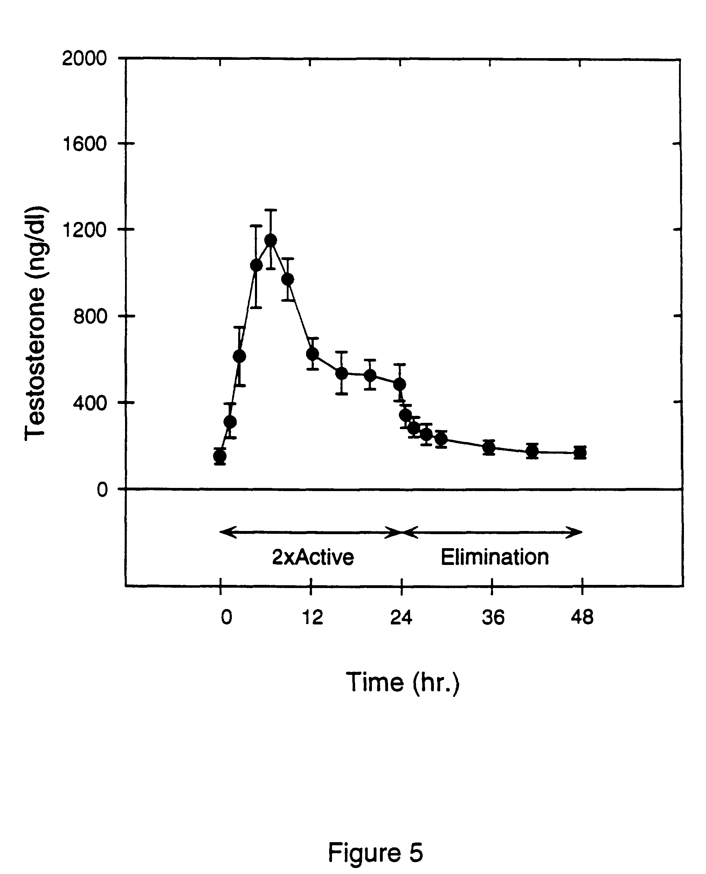

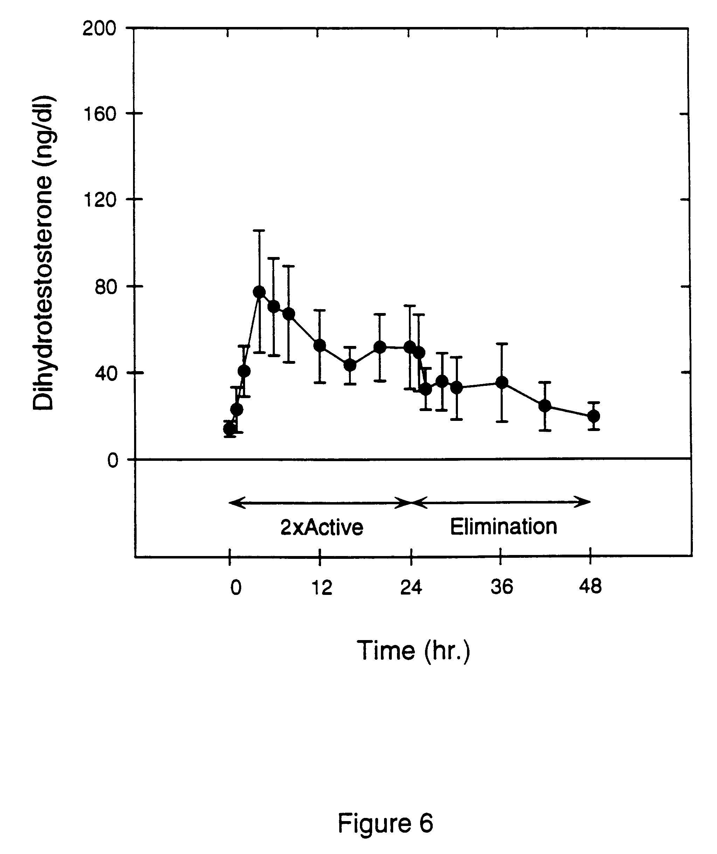

These composites (2 each) were placed on the lower backs of six hypogonadal men for 24 hr. Blood was sampled periodically over that period and their testosterone and DHT levels determined as in Example 9. Figures 5 and 6 report the results of these tests.

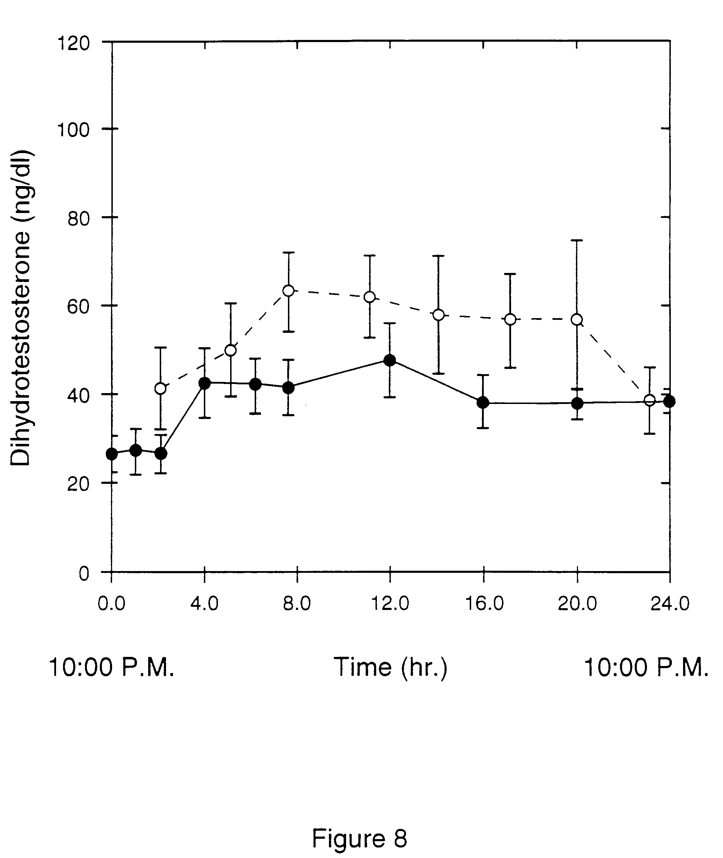

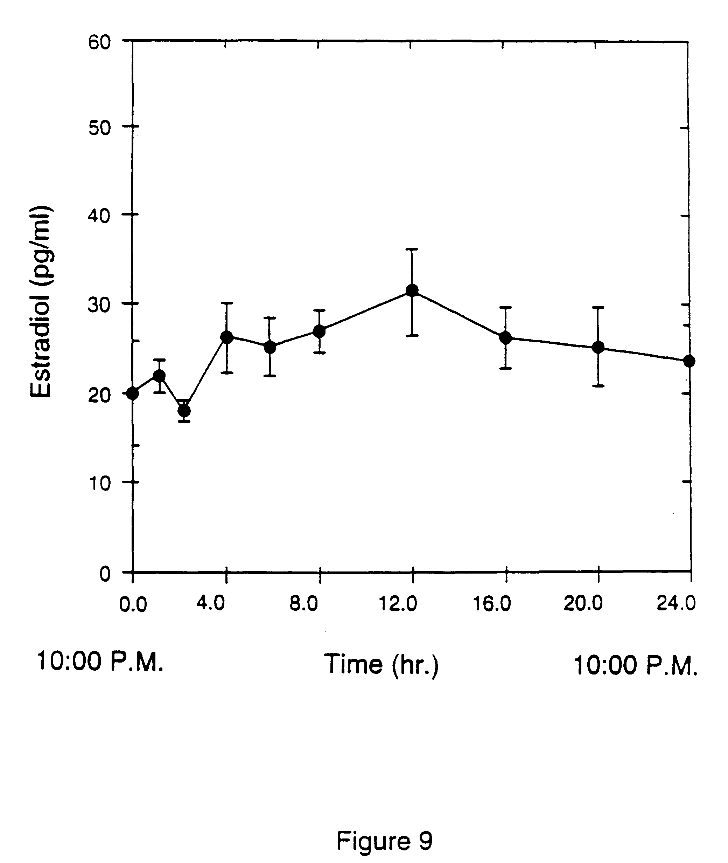

Figures 7, 8 and 9 depict average 24 hr plasma levels for testosterone, DHT, and E2 in five hypogonadal subjects following 28 days of continuous transdermal dosing as described above. Open circles in Figures 7 and 8 depict average testosterone and DHT levels determined in 12 normal male volunteers. These data demonstrate that physiological levels and circadian rhythms of testosterone and its active metabolites can be achieved and maintained using nonscrotal transdermal delivery systems according to the present invention.

## Claims

1. A device for administering by diffusion a hydrophobic drug transdermally to a patient for a prolonged period of time of at least one day, the drug having a solubility in water at room temperature of less than 50µg/ml and the device comprising:
(a) a reservoir comprising the drug dissolved in a carrier, the amount and solubility of the drug in the carrier defining a condition at the start of the period of subsaturation that is sufficient to provide a drug skin flux, over at least 60% of the period, that is at least 25% greater than the drug skin flux that would be provided if the carrier were saturated with the drug; and
(b) means for maintaining the reservoir in diffusional communication with the skin of the patient.

2. A device according to claim 1 where the hydrophobic drug is estradiol, progesterone, norethindrone acetate or medroxyprogesterone acetate.

3. A device according to claim 1 wherein the drug is testosterone, the skin is nonscrotal skin, and the drug skin flux substantially throughout the period is from 5 to 30 µg/cm²/hr.

4. A device according to claim 1 2 or 3 where the reservoir also contains a permeation enhancer.

5. A device according to any preceding claim wherein the prolonged period is from one to fourteen days.

6. A device according to any preceding claim where the solubility of the drug in the carrier is in the range of from 1 to 500 mg/ml.

7. A device according to any preceding claim wherein the concentration of drug in the carrier is from 20% to 80% of the saturation concentration of drug in the carrier substantially throughout the period.

8. A device according to any preceding claim wherein the means is the carrier and the carrier is an adhesive.

9. A device according to any preceding claim wherein the means is a basal adhesive layer underlying the reservoir, an adhesive overlay, or a ring of adhesive that is peripheral to the reservoir and is interconnected to the reservoir.

10. A device according to claim 3 wherein the carrier is a fluid.

11. A device according to claim 10 wherein the carrier is ethanol.

12. A device according to claim 11 wherein the permeation enhancer comprises glycerol monooleate and methyl laurate in combination with the ethanol.

13. A device according to any of claims 3 and 10 and 11 wherein the amount of testosterone in the reservoir is from 5 to 50 mg.

14. A device according to claim 12 or 13 wherein the reservoir contains 5% to 50% by volume glycerin.

15. A device as defined in any preceding claim for use in a method of treatment practised on the human body by therapy.

16. The use of a reservoir which comprises:
a hydrophobic drug having a solubility in water at room temperature of less than 50µg/ml dissolved in a carrier, the amount and solubility of the drug in the carrier defining a condition at the start of a prolonged period of time of at least one day of subsaturation that is sufficient to provide a drug skin flux, over at least 60% of the period, that is 25% greater than the drug skin flux that would be provided if the carrier were saturated with the drug;
for the manufacture of a medicament for administering, by diffusion, the drug transdermally.

## Patentansprüche

1. Vorrichtung zur Verabreichung durch transdermale Diffusion eines hydrophoben Arzneistoffs an einen Patienten über einen längeren Zeitraum von mindestens einem Tag, wobei der Arzneistoff eine Löslichkeit in Wasser bei Raumtemperatur von weniger als 50 µg/ml besitzt und wobei die Vorrichtung das Folgende umfasst:
(a) ein Reservoir, umfassend den in einem Träger gelösten Arzneistoff, wobei die Menge und Löslichkeit des Arzneistoffs in dem Träger am Beginn des Zeitraums einen Zustand der Untersättigung definieren, die ausreichend ist, um eine Arzneistoff-Hautdurchwanderung über mindestens 60 % des Zeitraums vorzusehen, das heisst, mindestens 25 % mehr als die Arzneistoff-Hautdurchwanderung, die vorgesehen würde, wenn der Träger mit dem Arzneistoff gesättigt wäre; und
(b) Mittel zur Aufrechterhaltung des Reservoirs in Diffusionsverbindung mit der Haut des Patienten.

2. Vorrichtung nach Anspruch 1, wobei der hydrophobe Arzneistoff Estradiol, Progesteron, Norethindronacetat oder Medroxyprogesteronacetat ist.

3. Vorrichtung nach Anspruch 1, wobei der Arzneistoff Testosteron ist, die Haut nicht-skrotale Haut ist und die Arzneistoff-Hautdurchwanderung im Wesentlichen während des gesamten Zeitraums 5 bis 30 µg/cm³/h beträgt.

4. Vorrichtung nach Anspruch 2 oder 3, wobei das Reservoir auch einen Permeationsverbesserer enthält.

5. Vorrichtung nach mindestens einem der vorhergehenden Ansprüche, wobei der längere Zeitraum ein bis vierzehn Tage beträgt.

6. Vorrichtung nach mindestens einem der vorhergehenden Ansprüche, wobei die Löslichkeit des Arzneistoffs in dem Träger im Bereich von 1 bis 500 mg/ml liegt.

7. Vorrichtung nach mindestens einem der vorhergehenden Ansprüche, wobei die Konzentration des Arzneistoffs in dem Träger 20 % bis 80 % der Sättigungskonzentration des Arzneistoffs in dem Träger ausmacht.

8. Vorrichtung nach mindestens einem der vorhergehenden Ansprüche, wobei das Mittel der Träger ist und der Träger ein Haftmittel ist.

9. Vorrichtung nach mindestens einem der vorhergehenden Ansprüche, wobei das Mittel eine dem Reservoir zugrundeliegende basale Haftschicht, ein haftender Überzug oder ein Ring aus Haftmittel ist, der peripher zu dem Reservoir angeordnet ist und mit dem Reservoir verbunden ist.

10. Vorrichtung nach Anspruch 3, wobei der Träger ein Fluid ist.

11. Vorrichtung nach Anspruch 10, wobei der Träger Ethanol ist.

12. Vorrichtung nach Anspruch 11, wobei der Permeationsverbesserer Glycerolmonooleat und Methyllaurat in Kombination mit dem Ethanol umfasst.

13. Vorrichtung nach mindestens einem der Ansprüche 3, 10 und 11, wobei die Menge an Testosteron in dem Reservoir 5 bis 50 mg beträgt.

14. Vorrichtung nach Anspruch 12 oder 13, wobei das Reservoir 5 bis 50 Vol.-% Glycerin enthält.

15. Vorrichtung, wie in mindestens einem der vorhergehenden Ansprüche für die Verwendung in einem Behandlungsverfahren definiert, das am menschlichen Körper durch eine Therapie praktiziert wird.

16. Verwendung eines Reservoirs, umfassend:
einen in einem Träger gelösten hydrophoben Arzneistoff mit einer Löslichkeit in Wasser bei Raumtemperatur von weniger als 50 µg/ml, wobei die Menge und Löslichkeit des Arzneistoffes in dem Träger einen Zustand der Untersättigung zu Beginn eines längeren Zeitraums von mindestens einem Tag definiert, der ausreichend ist, um eine Arzneistoff-Hautdurchwanderung über mindestens 60 % des Zeitraums vorzusehen, der um 25 % größer ist als die Arzneistoff-Hautdurchwanderung, die vorgesehen würde, wenn der Träger mit dem Arzneistoff gesättigt wäre;
für die Herstellung eines Medikaments zur Verabreichung des Arzneistoffs durch transdermale Diffusion.

## Revendications

1. Dispositif pour administrer par diffusion une substance médicamenteuse hydrophobe par voie transdermique à un patient pendant une période de temps prolongée, d'au moins un jour, la substance médicamenteuse ayant une solubilité dans l'eau à température ambiante inférieure à 50 µg/ml, et le dispositif comprenant :
(a) un réservoir comprenant la substance médicamenteuse dissoute dans un support, les quantité et solubilité de la substance médicamenteuse dans le support définissant une condition, au début de la période, de sous-saturation qui est suffisante pour fournir un flux cutané de la substance médicamenteuse, sur au moins 60 % de la période, qui est d'au moins 25 % supérieur au flux cutané de substance médicamenteuse qui serait obtenu si le support était saturé avec la substance médicamenteuse; et
(b) un moyen pour maintenir le réservoir en communication, permettant la diffusion, avec la peau du patient.

2. Dispositif selon la revendication 1, dans lequel la substance médicamenteuse hydrophobe est l'estradiol, la progestérone, l'acétate de noréthindrone ou l'acétate de médroxyprogestérone.

3. Dispositif selon la revendication 1, dans lequel la substance médicamenteuse est la testostérone, la peau est une peau non scrotale, et le flux cutané de substance médicamenteuse pendant sensiblement toute la période est de 5 à 30 µg/cm²/h.

4. Dispositif selon la revendication 2 ou 3, dans lequel le réservoir contient aussi un agent augmentant la perméation.

5. Dispositif selon l'une quelconque des revendications précédentes, dans lequel la période prolongée est d'un à quatorze jours.

6. Dispositif selon l'une quelconque des revendications précédentes, dans lequel la solubilité de la substance médicamenteuse dans le support est dans la gamme de 1 à 500 mg/ml.

7. Dispositif selon l'une quelconque des revendications précédentes, dans lequel la concentration de substance médicamenteuse dans le support est de 20 % à 80 % de la concentration à saturation de substance médicamenteuse dans le support pendant sensiblement toute la période.

8. Dispositif selon l'une quelconque des revendications précédentes, dans lequel le moyen est le support et le support est un adhésif.

9. Dispositif selon l'une quelconque des revendications précédentes, dans lequel le moyen est une couche adhésive basale sous le réservoir, une couche de recouvrement adhésive, ou un anneau d'adhésif qui est en périphérie du réservoir et est relié au réservoir.

10. Dispositif selon la revendication 3, dans lequel le support est un fluide.

11. Dispositif selon la revendication 10, dans lequel le support est l'éthanol.

12. Dispositif selon la revendication 11, dans lequel l'agent augmentant la perméation comprend du monooléate de glycérol et du laurate de méthyle combinés avec l'éthanol.

13. Dispositif selon l'une quelconque des revendications 3 et 10 et 11, dans lequel la quantité de testostérone dans le réservoir est de 5 à 50 mg.

14. Dispositif selon la revendication 12 ou 13, dans lequel le réservoir contient 5 % à 50 % en volume de glycérine.

15. Dispositif tel que défini dans l'une quelconque des revendications précédentes, pour l'utilisation dans une méthode de traitement pratiquée sur le corps humain par thérapie.

16. Utilisation d'un réservoir qui comprend : une substance médicamenteuse hydrophobe ayant une solubilité dans l'eau à température ambiante inférieure à 50 µg/ml dissoute dans un support, les quantité et solubilité de la substance médicamenteuse dans le support définissant, au début d'une période de temps prolongée, d'au moins un jour, une condition de sous-saturation qui est suffisante pour fournir un flux cutané de substance médicamenteuse sur au moins 60 % de la période, qui est de 25 % supérieur au flux cutané de substance médicamenteuse qui serait obtenu si le support était saturé avec la substance médicamenteuse ;
pour la fabrication d'un médicament pour administrer, par diffusion, la substance médicamenteuse par voie transdermique.
